(19) Europäisches Patentamt
European Patent Office
Office européen des brevets

(11) EP 3 882 023 A1

(12) EUROPEAN PATENT APPLICATION
published in accordance with Art. 153(4) EPC

(43) Date of publication:
**22.09.2021 Bulletin 2021/38**

(21) Application number: **20864323.9**

(22) Date of filing: **01.02.2020**

(51) Int Cl.:
***B32B 27/02*** (2006.01) ***B32B 27/36*** (2006.01)
***B32B 27/18*** (2006.01) ***B32B 27/40*** (2006.01)
***B32B 27/12*** (2006.01) ***B32B 27/08*** (2006.01)
***B32B 7/12*** (2006.01) ***B32B 33/00*** (2006.01)
***B29C 65/52*** (2006.01) ***A61L 31/12*** (2006.01)
***A61L 31/14*** (2006.01)

(86) International application number:
**PCT/CN2020/074148**

(87) International publication number:
**WO 2021/151259 (05.08.2021 Gazette 2021/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.01.2020 CN 202010077567**

(71) Applicant: **Sun Yat-Sen University**
**Guangzhou, Guangdong 510275 (CN)**

(72) Inventor: **The designation of the inventor has not yet been filed**

(74) Representative: **Sun, Yiming**
**HUASUN Patent- und Rechtsanwälte**
**Friedrichstraße 33**
**80801 München (DE)**

# (54) USE OF 3-(4-HYDROXYPHENYL)PROPIONIC ACID IN PREPARATION OF DRUGS FOR PREVENTING AND TREATING RESPIRATORY TRACT INFECTION

(57) The present invention relates to new medicine application of *p*-hydroxyphenylpropio nic acid. Experiments prove that *p*-hydroxyphenylpropionic acid has a remarkable pharmacological effect in inhibiting respiratory tract infection, particularly inhibiting pulmonary edema, inflammatory cell infiltration and cell apoptosis in lung injury and delaying lung function degradation of mice. Therefore, the present invention discloses the application of *p*-hydroxyphenylpropionic acid to prepare a medicine for preventing and treating respiratory tract infection, particularly pneumonia, pulmonary edema or pulmonary epithelial and/or endothelial cell necrosis in lung injury for the first time.

EP 3 882 023 A1

## Description

### TECHNICAL FIELD

The present invention relates to new medicine application of *p*-hydroxyphenylpropionic acid, particularly application of *p*-hydroxyphenylpropionic acid to prepare a medicine for preventing and treating respiratory tract infection.

### BACKGROUND

Respiratory tract infection is a common disease, which is divided into upper respiratory infection and lower respiratory tract infection. Severe illness may cause lung injury, particularly acute lung injury, which is an acute respiratory distress syndrome and is a common clinical severe acute disease. Pathological changes of the acute lung injury mainly include extensive inflammatory reaction in the lung, neutrophil recruitment, pulmonary edema, epithelial cell integrity breakage, microvascular permeability increase, gas exchange barrier and the like. According to statistics, the death rate of the acute lung injury may account for 34.9% of total hospital deaths. At present, there are no medicines approved by FDA and CFDA to treat acute lung injury. Therefore, it has an urgent necessity and broad market demand to develop a medicine for preventing and treating acute lung injury so as to reduce the clinical death rate of the acute lung injury.

p-Hydroxyphenylpropionic acid (HPPA) is an intermediate in the technical field of compound synthesis and has a structural formula:

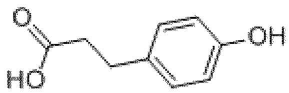

p-Hydroxyphenylpropionic acid may serve as an insecticide. Pharmacological experiments show that *p*-hydroxyphenylpropionic acid has an obvious protective effect on cobra poisoning in mice. Various esters produced by the reaction of *p*-hydroxyphenylpropionic acid and alcohols (such as ethanol, propanol, butanol and the like) may inhibit the growth of mucedine and are excellent preservatives. However, no literature has disclosed the related effect of *p*-hydroxyphenylpropionic acid in preventing and treating respiratory tract infection.

### SUMMARY

The present invention has been proved through experiments that in a lipopolysaccharide-induced mouse lung injury model, *p*-hydroxyphenylpropionic acid can obviously inhibit the pathological characteristics of pulmonary edema, inflammatory cell infiltration and cell apoptosis in lung injury and has a significant curative effect. In a chronic smoking model, p-hydroxyphenylpropionic acid can significantly delay the degradation of the lung function of the mice, significantly reduce the infiltration of neutrophil, lymphocytes and monocytes in mouse alveolar lavage fluid and significantly inhibit the increase of inflammatory factors in the lung tissue of the mice, thus inhibiting lung inflammation. As the *in-vitro* bacteriostasis effect, p-hydroxyphenylpropionic acid has a significant inhibition effect on *Klebsiella pneumoniae* and *Staphylococcus aureus.*

Therefore, the present invention discloses the new application of p-hydroxyphenylpropionic acid to prepare a medicine for preventing and treating respiratory tract infection, particularly pneumonia, pulmonary edema as well as pulmonary epithelial and endothelial cell necrosis in lung injury for the first time. The present invention further discloses application of p-hydroxyphenylpropionic acid to prepare a medicine for inhibiting *Klebsiella pneumoniae* and *Staphylococcus aureus.*

### DETAILED DESCRIPTION

Embodiment 1: evaluation on the pharmacodynamic effect of HPPA on lung injury

Experimental materials: lipopolysaccharide 055:B5, pentobarbital sodium, Memmert UFE400 oven, Mettler Toledo MS205DU hundred thousandth electronic balance.

Experimental animals: 42 SPF male Balb/c mice, aged 8 to 9 weeks old, with a weight of 18 g to 20 g, purchased from Ji'nan Pengyue Experimental Animal Breeding Ltd. Co. (Animal Permit No. SCXK (Lu) 20190003, and Occupancy Permit No. SYXK (Yue) 2016-0112).

Experimental method: the mice were acclimatized for 72 hours in an SPF animal room with constant temperature and constant humidity after purchase. The mice were randomly divided into four groups, that is, a blank control group (n=10), a model control group (n=10), a positive control medicine naringin group (n=11) and a p-hydroxyphenylpropionic

acid group (n=11). The naringin group (180 mg/kg) and the p-hydroxyphenylpropionic acid group (51.6 mg/kg) were continuously subjected to intragastric administration once a day, for three days respectively; and the blank control group and the model control group were administered intragastrically with equivoluminal normal saline.

The mice were anesthetized through intraperitoneal injection of 0.5 ml of pentobarbital sodium (65 mg/kg) three hours after the last intragastric administration. For the mice in the model group, the naringin group and the p-hydroxyphenylpropionic acid group, 50 μl of lipopolysaccharide 055:B5 solution (0.8 mg/ml) was slowly instilled into the nasal cavities of the mice until being completely absorbed, and a mouse acute lung injury model was established. The mice in the blank control group received nasal drops of the equivoluminal normal saline. The mice were killed by a cervical vertebra dislocation method 24 hours after the lipopolysaccharide modeling was performed, and the whole lung tissue of the mice was collected and put into a sterile centrifugal tube and was stored in liquid nitrogen within 30 seconds.

After the lung tissue stored in the liquid nitrogen was placed at room temperature for 30 minutes, a wet weight was weighed. A drying vessel was dried at 80°C for 24 hours and then was taken out and placed at room temperature for weighing. Then, the drying vessel was taken out after 1 hour of constant weight at 80°C and was placed at room temperature for weighing. After the weight of the drying vessel was constant, the lung tissue was placed in the drying vessel, was dried at 80°C for 48 hours and taken out, and was placed at room temperature for weighing. Then, the drying vessel was taken out after 1 hour of constant weight at 80°C and was placed at room temperature for weighing. The standard of constant weight was reached when the difference in weighing after two consecutive drying or ignition was less than 0.3 mg.

$$\text{Lung tissue dry-to-wet ratio} = \frac{\text{tissue wet weight (mg)}}{\text{constant weight in tissue drying vessel (mg)} - \text{constant weight of drying vessel (mg)}}$$

24 hours after the lipopolysaccharide modeling was performed, the mice were killed and about 400 mg of lung tissue was collected. 1 ml of PBS-10% Triton was added into the lung tissue to serve as a lysis solution for homogenization. After the protein concentration was measured by a BCA protein concentration kit, the sample loading quantity was adjusted to 50 μg of total protein and 100 μl of total volume, and the content of myeloperoxidase (MPO), the activity of the MPO and the activity of lactic dehydrogenase (LDH) in the lung tissue sample were measured by kits.

The data in the table are presented as mean ± standard deviation, and analysis of difference among multiple groups is realized by a one-way analysis of variance Beforroni method and SPSS 22.0 software. P value less than 0.01 is considered to have statistical difference.

Experimental results: the results are shown in Table 1. Compared with the mice in the blank control group, the mice in the model control group with acute lung injury (ALI) show significant pulmonary edema ($P$<0.01), accompanied by the increase of expression and activity of the MPO in the lung ($P$<0.01) and the increase of the activity of the LDH ($P$<0.01), indicating that the lipopolysaccharide-induced ALI model may effectively simulate three pathologic evidences of pulmonary edema, gathering infiltration of lymphocytes represented by neutrophil as well as pulmonary epithelial/endothelial cell necrosis in lung injury. Both the p-hydroxyphenylpropionic acid and the naringin may effectively inhibit the progress of lung inflammation in the ALI and alleviate pulmonary edema, local neutrophil infiltration and cell necrosis ($P$<0.01). Compared with the naringin, *p*-hydroxyphenylpropionic acid has a significant optimal effect in relieving inflammatory cell invasion and alleviating apoptosis and necrosis ($P$<0.01).

Table 1 Inhibition Effect on Lung Injury in Lipopolysaccharide-Induced Mice (mean ± standard deviation, n=6-8)

| Group | Lung Tissue Dry-to-wet Ratio | MPO Content in Lung (pg/ml) | MPO Activity in Lung (units/gprot) | LDH Activity in Lung (units/gprot) |
|---|---|---|---|---|
| Blank control | 4.49±0.25 | 166.57±39.94 | 0.948±0.069 | 173.65±15.17 |
| Model Control | 5.82±0.44 | 777.05±42.25 | 3.518±0.250 | 406.07±29.29 |
| Naringin group | 5.15±0.47** | 415.85±52.15** | 1.913±0.262** | 310.93±27.77** |

(continued)

| Group | Lung Tissue Dry-to-wet Ratio | MPO Content in Lung (pg/ml) | MPO Activity in Lung (units/gprot) | LDH Activity in Lung (units/gprot) |
|---|---|---|---|---|
| HPPA group | 4.90±0.52** | 324.09±36.33**,## | 1.756±0.190**, ## | 271.19±27.70**, ## |
| **, as compared with the model control group, $P$<0.01; and ##, as compared with the positive control medicine naringin group, **P**<0.01. | | | | |

Embodiment 2: improvement effect on chronic lung injury caused by smoking.

Experimental animals: SPF Balb/c mice, male, with a weight of 19 g to 23 g, purchased from Guangdong Medical Laboratory Animal Center (Guangzhou, China) Feeding conditions: 12-hour alternation of day and night, temperature 21°C, humidity 60%, free choice feeding.

Experimental instruments: smoking box (self-made stainless box 0.8m×0.8m×1m); hundred thousandth analytical balance (Germany Acculab Company, Model Number: ALC-210-4); 5430R high-speed centrifuge (Germany Eppendorf Company); porous ultramicro nucleic acid protein analyzer (USA Biotek Company, Model Number: Epoch); ultralow-temperature refrigerator (China Haier Company, Model Number: DW-86L486); vortex mixer (USA SI Company, Model Number: SI-0246; XT-2000IV fully-automatic animal blood analyzer (Japan Sysmex Company), etc.

Experimental groups: the animals were randomly divided into a blank group, a smoking model group, an HPPA low-dose group (15 mg/kg), an HPPA high-dose group (60 mg/kg) and a roflumilast group (5 mg/kg), with 8 animals in each group.

Modeling method and administration: after being adapted to feeding for one week, the animals were smoked and modeled, smoking once a day, 10 cigarettes per hour, continuously for 8 weeks. Each cigarette contains 11 mg of tar, 1.0 mg of nicotine and 13 mg of carbon monoxide. The normal group was not treated and the animals in the normal group were raised in a smoke-free environment. The administration groups began to take medicines in the third week, and were subjected to intragastric administration 1 hour before each smoking (the intragastric volume is 0.2 ml).

The mice were anesthetized through intraperitoneal injection of 0.25 ml of 4% chloral hydrate at the corresponding time points. The skin of the throat and neck of each mouse was cut to expose the trachea. A tracheal catheter was inserted into an opening formed under the thyroid cartilage and the opening was ligated with a cotton thread. The intubated mice were put into a PET small animal lung function analysis system body tracing box, and the quasi-static lung compliance (Cchord) was measured. Meanwhile, forced expiratory volume (FEV50) within 50 ms and the forced vital capacity (FVC) were measured to calculate FEV50/FVC.

8 mice in each group were killed in a cervical vertebra dislocation manner at the corresponding time points, the abdomen was transversely opened, the diaphragmatic pleura was cut, then the ribs on two sides were cut upwards until the whole front chest was opened, and the whole lung was lifted with forceps and cut off, was packaged in a small sealed bag and was stored in a refrigerator at -80°C.

The throat skin and muscle were cut, the trachea was separated carefully, an opening was formed under the thyroid cartilage with ophthalmic scissors, tracheal intubation was conducted by a 5 mL injection needle and the opening was tightened. After the chest cavity was opened, 0.5 mL of normal saline was injected from the tracheal intubation position for whole lung lavage continuously for three times, the bronchoalveolar lavage fluid (BALF) of three times were mixed, centrifugation was conducted at 3000 rpm, the cell pellet was resuspended with 300 μL of PBS buffer solution containing 2% fetal calf serum, and leukocytes were classified and counted by a fully-automatic five-category animal blood analyzer to calculate the number of the neutrophil, lymphocytes and monocytes.

The lung tissue and the tissue homogenate were mixed in a ratio of 1mg:10μL, the lung tissue was uniformly ground by a glass homogenizer to prepare 10% lung tissue homogenate, the total protein content of the lung tissue homogenate was measured by a BCA method, the contents of TNFα, IL-6, $LTB_4$, IL-1β and IL-18 in the lung tissue homogenate were measured by an ELISA method. Operation and measurement were conducted according to the specification of the kit, and the content of each cell factor was expressed as pg/mg protein.

The experimental results were shown in Table 2 to Table 4.

1. The HPPA can significantly inhibit the reduction of the Cchord and FEV50/FVC of the mice and delay the degradation of the lung function of the mice. The experimental result is shown in Table 2.

Table 2 Effect on the Lung Function of the Chronic Smoked Mice

| Group | Cchord (mL/cm H2O) | FEV50/FVC |
|---|---|---|
| Blank group | 0.034±0.0045 | 0.39±0.065 |
| Model group | 0.052±0.0013** | 0.227±0.048** |
| HPPA low-dose | 0.0448±0.0041 | 0.360±0.039## |
| group | | |
| HPPA high-dose group | 0.041±0.0029## | 0.333±0.040## |
| Roflumilast | 0.042±0.0054# | 0.367±0.042## |
| As compared with the blank group, * shows P<0.05 and ** shows P<0.01; and as compared with the model group, # shows *P*<0.05 and ## shows P<0.01. | | |

2. The HPPA can significantly reduce the infiltration of the neutrophil, lymphocytes and monocytes in the alveolar lavage fluid of the chronic smoked mice. The experimental result is shown in Table 3.

Table 3 Leukocyte Count of Alveolar Lavage Fluid of the Chronic Smoked Mice

| Group | Neutrophil ($10^5$/mL) | Lymphocyte ($10^5$/mL) | Monocyte ($10^5$/mL) |
|---|---|---|---|
| Blank group | 0.171±0.0756 | 0.229±0.0756 | 1.871±0.304 |
| Model group | 1.200±0.316** | 1.367±0.398** | 3.886±0.524** |
| HPPA low-dose group | 1.129±0.287 | 1.317±0.117 | 3.871±0.544 |
| HPPA high-dose group | 0.743±0.190## | 1.014±0.107# | 3.100±0.554## |
| Roflumilast group | 0.729±0.180## | 0.914±0.227## | 3.014±0.319## |
| As compared with the blank group, * shows P<0.05 and ** shows P<0.01; and as compared with the model group, # shows *P*<0.05 and ## shows P<0.01. | | | |

3. The HPPA can significantly inhibit the increase of the TNFα, IL-6, $LTB_4$, IL-1β and IL-18 levels in the lung tissue of the chronic smoked mice, thereby inhibiting inflammation. The experimental result is shown in Table 4.

Table 4 Effect on Inflammatory Factors in Lung Tissue of Chronic Smoked Mice

| Group | TNF-α (pg/mL) | IL-6 (pg/mL) | $LTB_4$ (pg/mL) | IL-1β (pg/mL) | IL-18 (pg/mL) |
|---|---|---|---|---|---|
| Blank group | 89.88±11.23 | 968.3±127.1 | 6666±1235 | 32.23±6.231 | 82.14±11.23 |
| Model group | 151.5±18.98 ** | 1520±159.2* * | 9903±971.1 ** | 55.23±6.334 ** | 188.4±10.45 ** |
| HPPA low-dose group | 126.3±10.34 # | 1483±300.3 | 8344±1356# | 47.18±6.822 | 180.1±6.667 |
| HPPA high-dos e group | 92.45±13.24 ## | 1027±90.56# # | 7241±1024# # | 35.27±6.509 ## | 157.4±9.098 ## |
| Roflumila st group | 102.4±17.34 ## | 963.2±188.8 ## | 6809±670.8 ## | 34.02±7.098 ## | 143.4±18.34 ## |
| As compared with the blank group, * shows *P*<0.05 and ** shows *P*<0.01.<br>As compared with the model group, # shows *P*<0.05 and ## shows *P*<0.01. | | | | | |

Embodiment 3: research on antibacterial activity *in vitro*

Experimental instruments: hundred thousandth electronic analysis balance (BP211D, Germany Satorius Company), high-pressure and high-temperature sterilizer (GR 60DA), ultrasonic oscillation cleaner (KQ-250DE, Kunshan

Ultrasonic Instrument Ltd. Co.), pipette (France Gilson), Mcfarland turbidity comparator (VITEK 2 DensiCHEK Plus, 27208 densitometer densichek kit 220), Mcfarland turbidimetric tube (France Mérieux Company), bacterial constant temperature incubator (HPX-9162MBE electro-heating constant-temperature cultivator, Changzhou Nocchi Instrument Ltd. Co.), sterile 96-well plate (JET BIOFIL), 90mm Columbia blood agar culture medium (43041COLUMBIA 5% SHEEP BL, France Mérieux Company).

Experimental strains: 10 strains of experimental *Staphylococcus aureus* (No. S1-S10) and 12 strains of *Klebsiella pneumoniae* (No. K1-K12) were provided by the Laboratory Department of the Affiliated First Hospital of Sun Yat-sen University.

Experimental method:

Recovery and passage of bacteria: after an incubating loop was sterilized at high temperature, the blood agar culture medium was coated with the cryopreserved and collected strains in the form of plate streaking and was reversely put into a bacteriological incubator; and after culture was conducted at 37°C for 24 hours, a new blood plate medium was coated with the picked single colony, and culture was continuously conducted at 37°C for 24 hours.

Antibacterial experiment *in vitro:* the antibacterial experiment *in vitro* was designed with reference to a broth microdilution method according to the standard of American Clinical Laboratory Standardization Institute (CLSI). The HPPA was subjected to doubling dilution with a culture medium to obtain a series of medicine concentration, and then was added into each well of the sterile 96-well plate respectively. A blank control well was set, in which normal saline rather than medicine liquid was added. 50 μl was added in each well in the experiment group and the control group. Bacteria were picked and normal saline was added into the bacteria, Mcfarland turbidity was adjusted to 0.5 after the solution was oscillated and mixed uniformly, the solution was diluted with the culture medium by 100 times and was added into each well of the sterile 96-well plate, 50 μl in each well, that is, the final incubation amount is $1.0*10^6$ CFU/ml. After uniform mixing and 20-hour cultivation at 37°C, the turbid degree of the culture solution in each well and the precipitation in each well were detected, and the medicine concentration corresponding to the clear pore is the minimum inhibitory concentration (MIC) of the medicine. The results are shown in Table 5 and Table 6.

Table 5 Result of Antibacterial Experiment *in Vitro* of *Klebsiella pneumoniae*

| Strain Number | MIC (μg/ml) |
|---|---|
| K1 | 128 |
| K2 | 128 |
| K3 | 128 |
| K4 | 128 |
| K5 | 128 |
| K6 | 64 |
| K7 | 128 |
| K8 | 64 |
| K9 | 64 |
| K10 | 64 |
| K11 | 64 |
| K12 | 64 |

Table 6 Result of Antibacterial Experiment *in Vitro* of *Staphylococcus aureus*

| Bacterial Number | MIC μg/mL |
|---|---|
| S1 | 64 |
| S2 | 64 |
| S3 | 64 |

(continued)

| Bacterial Number | MIC μg/mL |
|---|---|
| S4 | 64 |
| S5 | 64 |
| S6 | 64 |
| S7 | 64 |
| S8 | 64 |
| S9 | 64 |
| S10 | 64 |

**Claims**

1. Application of p-hydroxyphenylpropionic acid to prepare a medicine for preventing and treating respiratory tract infection.

2. Application of p-hydroxyphenylpropionic acid to prepare a medicine for preventing and treating lung injury.

3. Application of p-hydroxyphenylpropionic acid to prepare a medicine for preventing and treating pneumonia.

4. Application of p-hydroxyphenylpropionic acid to prepare a medicine for preventing and treating acute pneumonia.

5. Application of *p*-hydroxyphenylpropionic acid to prepare a medicine for preventing and treating chronic pneumonia.

6. Application of p-hydroxyphenylpropionic acid prepare a medicine for preventing and treating pulmonary edema.

7. Application of p-hydroxyphenylpropionic acid to prepare a medicine for preventing and treating pulmonary epithelial or endothelial cell necrosis.

8. Application of *p*-hydroxyphenylpropionic acid to prepare a medicine for inhibiting *Klebsiella pneumoniae.*

9. Application of *p*-hydroxyphenylpropionic acid to prepare a medicine for inhibiting staphylococcus aureus.

10. The application according to claims 1 to 9, wherein the medicine is prepared into a clinically acceptable preparation.

## INTERNATIONAL SEARCH REPORT

International application No.
**PCT/CN2020/074148**

**A. CLASSIFICATION OF SUBJECT MATTER**

A61K 31/192(2006.01)i; A61P 31/04(2006.01)i; A61P 11/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, CNTXT, DWPI, WOTXT, USTXT, EPTXT, CNKI, Web of Science, STN, 百度学术, 读秀学术: 中山大学, 吴灏, 陈韬彬, 王永刚, 苏薇薇, 对羟基苯丙酸, 柚皮苷, 柚苷, 柑橘甙, 异橙皮苷, 呼吸道, 肺炎, 肺损伤, 肺水肿, 肺上皮细胞, 肺内皮细胞, 细胞坏死, 感染, 抗菌, 抑菌, 克雷伯, 葡萄球菌, AURANTIIN, HPPA, hydroxyphenyl, ropionic acid, Respiratory tract, pneumonia, lung injury, pulmonary edema, lung epithelial cell+, lung endothelial cell+, Klebsiella, Staphylococcus, 501-97-3

**C. DOCUMENTS CONSIDERED TO BE RELEVANT**

| Category* | Citation of document, with indication, where appropriate, of the relevant passages | Relevant to claim No. |
|---|---|---|
| X | CUEVA, Carolina et al.,. "Antimicrobial activity of phenolic acids against commensal, probiotic and pathogenic bacteria," *RESEARCH IN MICROBIOLOGY,* Vol. 161, 31 December 2010 (2010-12-31), pp. 372-382 | 9-10 |
| A | 李沛波 等 (LI, Peibo et al.). "以化橘红为基源的一类新药柚皮苷的临床前研究 (The Pre-Clinical Studies of Naringin, An Innovative Drug, Derived from Citri Grandis Exocarpium(Huajuhong))" *中山大学学报 (自然科学版) (Acta Scientiarum Naturalium Universitatis Sunyatseni),* Vol. 54, No. 6, 30 November 2015 (2015-11-30), pp. 1-5 | 1-10 |
| A | CN 108503674 A (JI'AN COLLEGE) 07 September 2018 (2018-09-07) description, paragraph 3 | 1-10 |
| A | CN 103833809 A (LI, Yushan) 04 June 2014 (2014-06-04) description, paragraph 4 | 1-10 |
| A | CN 1468602 A (SUN YAT-SEN UNIVERSITY) 21 January 2004 (2004-01-21) description page 1 paragraph 2, page 2 paragraph 2 from the bottom | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 October 2020** | **03 November 2020** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

INTERNATIONAL SEARCH REPORT

International application No.
PCT/CN2020/074148

C. DOCUMENTS CONSIDERED TO BE RELEVANT

| Category* | Citation of document, with indication, where appropriate, of the relevant passages | Relevant to claim No. |
|---|---|---|
| A | CN 109593110 A (HUAZHONG AGRICULTURAL UNIVERSITY) 09 April 2019 (2019-04-09) description, paragraph 2 | 1-10 |
| A | CN 1733125 A (JIANGXI TIANKE PHARMACEUTICAL DEVELOPMENT CO., LTD.) 15 February 2006 (2006-02-15) entire document | 1-10 |
| A | GAO, Sen et al.,. "Antitussive Effect of Naringin on Experimentally," *Planta Med,* Vol. 77, No. 1, 31 December 2011 (2011-12-31), pp. 16-21 | 1-10 |

Form PCT/ISA/210 (second sheet) (January 2015)

**INTERNATIONAL SEARCH REPORT**
**Information on patent family members**

International application No.
**PCT/CN2020/074148**

| Patent document cited in search report | Publication date (day/month/year) | Patent family member(s) | Publication date (day/month/year) |
|---|---|---|---|
| CN 108503674 A | 07 September 2018 | CN 108503674 B | 21 April 2020 |
| CN 103833809 A | 04 June 2014 | None | |
| CN 1468602 A | 21 January 2004 | CN 1179726 C | 15 December 2004 |
| CN 109593110 A | 09 April 2019 | None | |
| CN 1733125 A | 15 February 2006 | CN 1733125 B | 28 April 2010 |

Form PCT/ISA/210 (patent family annex) (January 2015)